# EUROPEAN PATENT APPLICATION

(11) **EP 4 215 833 A1**
(43) Date of publication of application: **26.07.2023**
(21) Application number: 21875643.5
(22) Date of filing: 28.09.2021
(51) Int. Cl.: F24F 11/66, F24F 110/10, F24F 120/10, A61B 5/16

(54) **TEMPERATURE ESTIMATION DEVICE, AIR CONDITIONING CONTROL DEVICE, AND AIR CONDITIONING CONTROL SYSTEM**

(30) Priority: 30.09.2020 JP 2020166144
(71) Applicant: Daikin Industries, Ltd., Osaka-shi, Osaka 530-0001 (JP); OSAKA UNIVERSITY, Suita-shi Osaka 565-0871 (JP)
(72) Inventor: TSUBOI, Chiaki, Osaka-shi, Osaka 530-0001 (JP); HORI, Shouta, Osaka-shi, Osaka 530-0001 (JP); KATO, Takafumi, Osaka-shi, Osaka 530-0001 (JP); KAMIMURA, Mayo, Osaka-shi, Osaka 530-0001 (JP)
(74) Representative: Hoffmann Eitle
(86) International application number: PCT/JP2021/035702
(87) International publication number: WO 2022/071342

(57) **Abstract**

The derivation unit (31) determines the stability of a parasympathetic nerve activity of the sleeper (P) for each environment temperature, and derives a correspondence relationship between the environment temperature and the stability of the parasympathetic nerve activity of the sleeper (P). An estimation unit (32) estimates an appropriate value (T1) of the environment temperature suitable for the sleeper (P) based on the correspondence relationship derived by the derivation unit (31).

## Description

### Technical Field

The present disclosure relates to a temperature estimation device, an air conditioning control device, and an air conditioning control system.

### Background Art

PTL 1 discloses a sleep environment temperature control device that controls a sleep environment temperature. The device includes biological signal detection means, sleep depth determination means, and temperature control means. The biological signal detection means detects a biological signal of a user in a non-invasive and non-constrained manner. The sleep depth determination means determines a sleep depth of the user based on time-series data of the biological signal detected by the biological signal detection means. The temperature control means controls, based on the sleep depth determined by the sleep depth determination means, a sleep environment temperature by controlling a control subject device having a temperature adjustment function.

### Citation List

### Patent Literature

PTL 1: Japanese Unexamined Patent Publication No. 2019-24628

### Summary of Invention

### Technical Problem

However, it is difficult to estimate an appropriate value of the environment temperature suitable for the sleeper. For example, the appropriate value of the environment temperature differs for each sleeper.

### Solution to Problem

A first aspect of the present disclosure relates to a temperature estimation device including: a derivation unit (31) configured to determine a stability of a parasympathetic nerve activity of a sleeper (P) for each environment temperature, and to derive a correspondence relationship between the environment temperature and the stability of the parasympathetic nerve activity of the sleeper (P); and an estimation unit (32) configured to estimate an appropriate value (T1) of the environment temperature suitable for the sleeper (P) based on the correspondence relationship derived by the derivation unit (31).

In the first aspect, it is possible to estimate the appropriate value (T1) of the environment temperature suitable for the sleeper (P) based on the correspondence relationship between the environment temperature and the stability of the parasympathetic nerve activity of the sleeper (P).

A second aspect of the present disclosure is the temperature estimation device according the first aspect, wherein the derivation unit (31) determines the stability of the parasympathetic nerve activity of the sleeper (P) based on the parasympathetic nerve activity of the sleeper (P) in a non-REM sleep period (P11, P21, P31, P41) of the sleeper (P).

In the second aspect, it is possible to accurately determine the stability of the parasympathetic nerve activity of the sleeper (P) while avoiding a REM sleep period (P12, P22, P32, P42) in which the autonomic nerves of the sleeper (P) tend to be unstable. Accordingly, it is possible to accurately estimate the appropriate value (T1) of the environment temperature suitable for the sleeper (P).

A third aspect of the present disclosure is the temperature estimation device according to the first or second aspect, wherein the derivation unit (31) derives the correspondence relationship between the environment temperature and the stability of the parasympathetic nerve activity of the sleeper (P) for each sleep stage of the sleeper (P).

In the third aspect, it is possible to estimate the appropriate value (T1) of the environment temperature suitable for the sleeper (P) for each sleep stage of the sleeper (P).

A fourth aspect of the present disclosure relates to an air conditioning control device for controlling an air conditioner (15) that performs air conditioning of a space in which a sleeper (P) is present, the air conditioning control device including: the temperature estimation device according to any one of the first to third aspects; and an air conditioning control unit (35) configured to control the air conditioner (15) based on the appropriate value (T1) of the environment temperature estimated by the temperature estimation device.

In the fourth aspect, it is possible to appropriately control the air conditioner (15) based on the appropriate value (T1) of the environment temperature suitable for the sleeper (P).

A fifth aspect of the present disclosure is the air conditioning control device according to the fourth aspect, wherein the air conditioning control unit (35) controls the air conditioner (15) so that the environment temperature becomes the appropriate value (T1), and then, if a predetermined condition is satisfied, controls the air conditioner (15) so that the environment temperature becomes higher than the appropriate value (T1).

In the fifth aspect, it is possible to suppress excessive cooling of the sleeper (P) after the predetermined condition is satisfied.

A sixth aspect of the of the present disclosure is the air conditioning control device of the fifth aspect, wherein the condition includes at least one of a first condition that a first REM sleep period (P12) starts after the sleeper (P) falls asleep, a second condition that an amount of decrease in the stability of the parasympathetic nerve activity of the sleeper (P) exceeds a predetermined reference amount after the sleeper (P) falls asleep, a third condition that a predetermined time elapses after the sleeper (P) enters a bed, or a fourth condition that a predetermined time elapses after the sleeper (P) falls asleep.

In the sixth aspect, it is possible to suppress excessive cooling of the sleeper (P) after at least one of the first to fourth conditions is satisfied.

A seventh aspect of the present disclosure is the air conditioning control device according to the fourth aspect, wherein the air conditioning control unit (35) controls the air conditioner (15) so that the environment temperature changes at a second time point (t2) before a first time point (t1) at which a deep temperature of the sleeper (P) is to be changed.

In the seventh aspect, it is possible to control the environment temperature in consideration of a time difference before a change in the environment temperature affects the deep temperature of the sleeper (P). This makes it possible to appropriately control the deep temperature of the sleeper (P).

An eighth aspect of the present disclosure relates to an air conditioning system, the air conditioning system including: an air conditioner (15) that performs air conditioning of a space in which a sleeper (P) is present; and the air conditioning control device according to any one of the fourth to seventh aspects that controls the air conditioner (15).

In the eighth aspect, it is possible to appropriately control the air conditioner (15) based on the appropriate value (T1) of the environment temperature suitable for the sleeper (P).

### Brief Description of Drawings

Fig. 1 is a schematic diagram illustrating a configuration of an air conditioning system of a first embodiment.
Fig. 2 is a flow chart illustrating an appropriate temperature estimation process.
Fig. 3 is a graph illustrating a time-series data signal of a peak interval (RR interval) of a heartbeat waveform.
Fig. 4 is a graph illustrating a signal indicating a temporal change in amplitude of a frequency component correlated with a parasympathetic nerve activity of a sleeper.
Fig. 5 is a graph showing an example of a correspondence relationship between an environment temperature and a parasympathetic nerve activity duration ratio.
Fig. 6 is a graph showing another example of the correspondence relationship between the environment temperature and the parasympathetic nerve activity duration ratio.
Fig. 7 is a schematic diagram illustrating a configuration of an air conditioning system of a second embodiment.
Fig. 8 is a graph for explaining sleep stages of a sleeper.
Fig. 9 is a graph showing an example of air conditioning control in a fourth embodiment.
Fig. 10 is a schematic diagram illustrating a configuration of an air conditioning system of a fifth embodiment.
Fig. 11 is a graph showing an example of air conditioning control in the fifth embodiment.
Fig. 12 is a graph showing another example of air conditioning control in the fifth embodiment.

### Description of Embodiments

Hereinafter, embodiments will be described in detail with reference to the drawings. In the drawings, the same or corresponding portions are denoted by the same reference numerals, and description thereof will not be repeated.

### (First Embodiment)

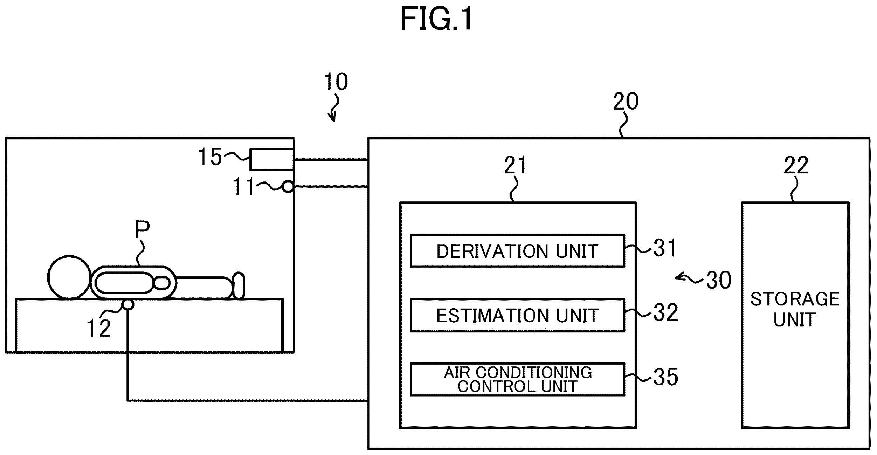

Fig. 1 illustrates a configuration of an air conditioning system (10) of a first embodiment. The air conditioning system (10) includes an air conditioner (15) and an air conditioning control device (20). The air conditioner (15) performs air conditioning of a space in which a sleeper (P) is present. Specifically, the air conditioner (15) adjusts the temperature of the space in which the sleeper (P) is present.

Further, the air conditioning system (10) is provided with various sensors. The various sensors output detection signals indicating detection results. In this example, the air conditioning system (10) is provided with an environment temperature sensor (11) and a biological sensor (12).

The environment temperature sensor (11) detects an environment temperature that is the temperature of a surrounding environment of the sleeper (P). In this example, the environment temperature is the temperature of the air in the space in which the sleeper (P) is present.

The biological sensor (12) detects a biological signal derived from a biological activity of the sleeper (P). In this example, the biological signal of the sleeper (P) is a heartbeat signal of the sleeper (P).

For example, the biological sensor (12) is a body motion sensor. The body motion sensor detects a body motion signal derived from the biological activity of the sleeper (P), and processes the detected body motion signal to output a biological signal of the sleeper (P).

Specifically, the body motion sensor includes a tube, a sensor unit, and a signal processing unit. The tube is a flexible tube made of resin and is disposed across the sleeper (P). The internal pressure of the tube varies in accordance with the body motion of the sleeper (P). The sensor unit is provided at one end of the tube and converts a change in the internal pressure of the tube into an electric signal. For example, the sensor unit is a microphone. The electric signal output from the sensor unit is a body motion signal. The signal processing unit processes the body motion signal output from the sensor unit to extract a biological signal (in this example, a heartbeat signal) of the sleeper (P) from the body motion signals.

### [Air conditioning control device]

The air conditioning control device (20) controls the air conditioner (15). The air conditioning control device (20) includes a control unit (21) and a storage unit (22).

The control unit (21) controls the operation of the air conditioning system (10) based on detection signals of various sensors provided in the air conditioning system (10). For example, the control unit (21) includes a processor and a memory that is electrically connected to the processor and stores a program and information for operating the processor.

The storage unit (22) stores various kinds of data and information.

The control unit (21) includes a derivation unit (31), an estimation unit (32), and an air conditioning control unit (35). Specifically, the processor of the control unit (21) executes various programs stored in the memory, whereby the control unit (21) functions as the derivation unit (31), the estimation unit (32), and the air conditioning control unit (35). The derivation unit (31) and the estimation unit (32) are elements constituting a temperature estimation device (30) that estimates an appropriate value of the environment temperature suitable for the sleeper (P).

The derivation unit (31) determines the stability of a parasympathetic nerve activity of the sleeper (P) for each environment temperature, and derives a correspondence relationship between the environment temperature and the stability of the parasympathetic nerve activity of the sleeper (P). As the stability of the parasympathetic nerve activity of the sleeper (P) becomes higher, the sleeper (P) becomes more relaxed, and the quality of the sleep of the sleeper (P) tends to be better.

The estimation unit (32) estimates an appropriate value (T1) of the environment temperature suitable for the sleeper (P) based on the correspondence relationship between the environment temperature and the stability of the parasympathetic nerve activity of the sleeper (P), the correspondence relationship being derived by the derivation unit (31).

The air conditioning control unit (35) controls the air conditioner (15) based on the appropriate value (T1) of the environment temperature estimated by the estimation unit (32).

### [Temperature estimation process]

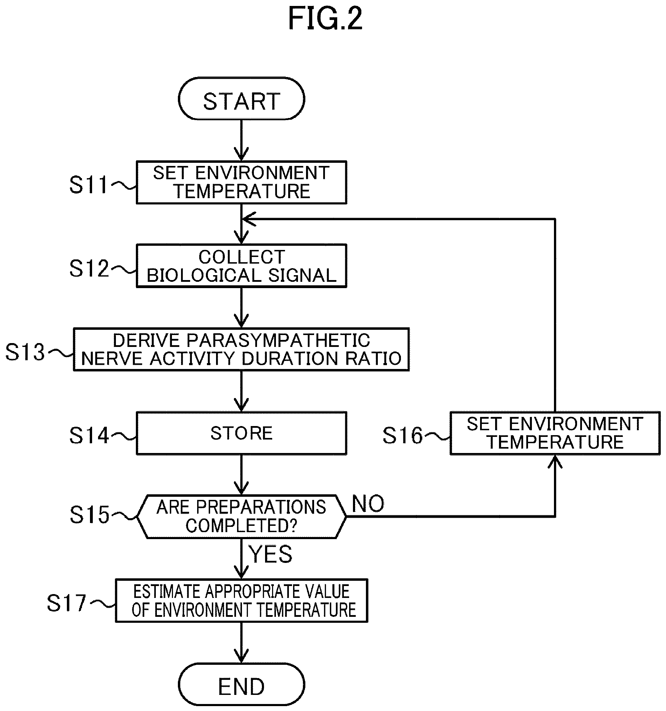

Next, a temperature estimation process of the first embodiment will be described with reference to Fig. 2.

### <Step (S11)>

First, the derivation unit (31) sets the environment temperature to a predetermined initial value before the sleeper (P) enters the bed (for example, 30 minutes before the entry to the bed). For example, the initial value of the environment temperature is an appropriate value of the environment temperature obtained from an experiment result for each of a plurality of sleepers (P). Specifically, the initial value of the environment temperature may be 26°C. The presence or absence of the entry to the bed of the sleeper (P) can be detected based on the presence or absence of the biological signal of the sleeper (P).

Specifically, the derivation unit (31) sets a target environment temperature, which is a target value of the environment temperature, to a predetermined initial value before the sleeper (P) enters the bed. The air conditioning control unit (35) controls the air conditioner (15) so that the environment temperature detected by the environment temperature sensor (11) becomes the target environment temperature set by the derivation unit (31).

### <Step (S12)>

Next, the derivation unit (31) collects the biological signal detected by the biological sensor (12) in a sleep period which is the period from when the sleeper (P) enters the bed to when the sleeper (P) awakens. In this example, a heartbeat signal of the sleeper (P) during the sleep period is collected.

### <Step (S13)>

Next, the derivation unit (31) derives a duration ratio of the parasympathetic nerve activity of the sleeper (P) based on the biological signal of the sleeper (P) collected in step (S12). The duration ratio of the parasympathetic nerve activity of the sleeper (P) is an example of the stability of the parasympathetic nerve activity of the sleeper (P).

In this example, the derivation unit (31) derives the duration ratio of the parasympathetic nerve activity of the sleeper (P) as follows.

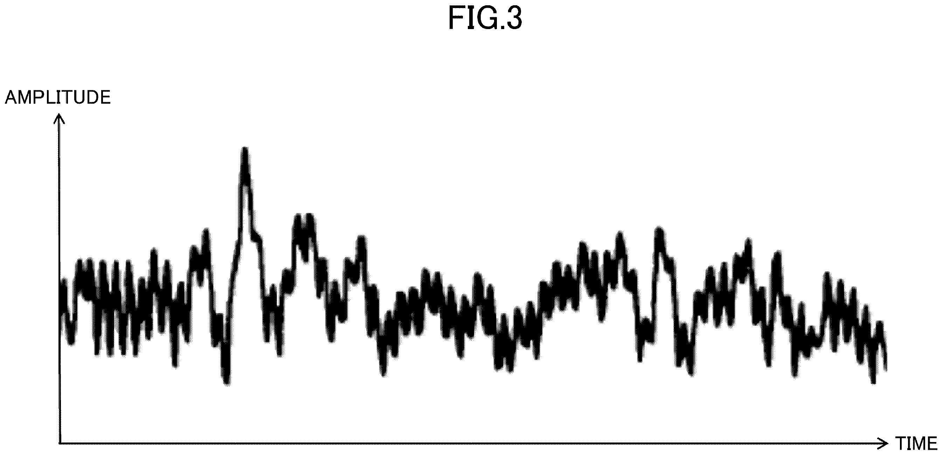

First, the derivation unit (31), based on the heartbeat signal of the sleeper (P) collected in step (S12) in the sleep period (period from the entry to the bed of the sleeper (P) to the awakening), generates a time-series data signal of a peak interval (RR interval) of a heartbeat waveform, as shown in Fig. 3. The peak interval is an interval between R waves of the heartbeat waveform.

Next, the derivation unit (31) extracts a signal of a frequency component correlated with the parasympathetic nerve activity of the sleeper (P) from the time-series data signals of the peak interval. For example, the frequency component correlated with the parasympathetic nerve activity of the sleeper (P) is a component in a frequency band of 0.15 to 0.4 Hz.

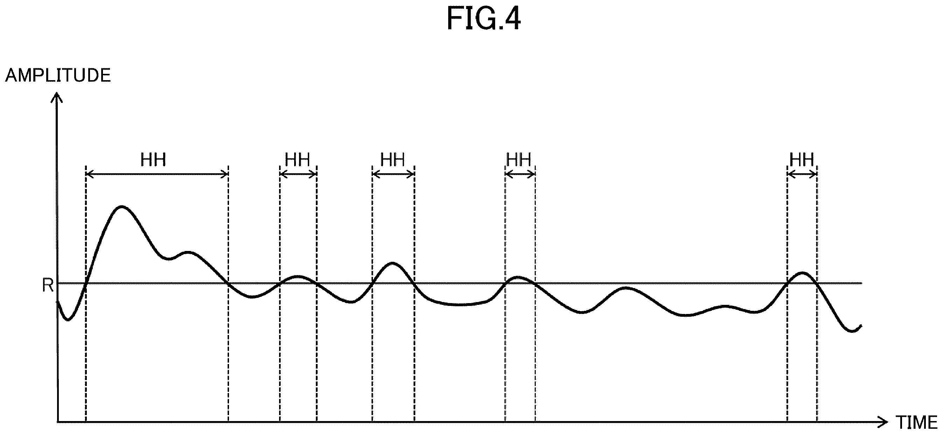

Next, the derivation unit (31) performs calculation by a complex demodulation method (CDM) on the signal of the frequency component correlated with the parasympathetic nerve activity of the sleeper (P), to generate a signal (hereafter referred to as a "feature signal") indicating a temporal change in amplitude of the frequency component correlated with the parasympathetic nerve activity of the sleeper (P), as shown in Fig. 4.

While the derivation of the feature signal by CDM has been described, this is an example and not a limitation. For example, the derivation unit (31) may derive the feature signal by continuously performing frequency analysis such as fast Fourier transform (FFT) on the signal of the frequency component correlated with the parasympathetic nerve activity of the sleeper (P).

For the above processing, it is possible to adopt well-known techniques disclosed in, e.g., the non-patent literature "Analysis of autonomic cardiovascular regulation during dynamic and isometric exercises by complex demodulation of heart rate and blood pressure variabilities", and the patent literature "Japanese Unexamined Patent Publication No. 2004-81513".

Next, the derivation unit (31) compares the amplitude value of the feature signal with a predetermined threshold (R), and detects a high-level period (HH) that is a period during which the amplitude value of the feature signal exceeds the threshold (R) in the sleep period (period from when the sleeper (P) enters the bed to when the sleeper (P) awakens). Then, the derivation unit (31) derives the ratio of the total period of the high-level periods (HH) to the sleep period as "the duration ratio of the parasympathetic nerve activity of the sleeper (P)".

### <Step (S14)>

Next, the derivation unit (31) stores identification information for identifying the sleeper (P), the environment temperature set in step (S11) (or step (S16)), and the duration ratio of the parasympathetic nerve activity of the sleeper (P) derived in step (S13) in association with each other in the storage unit (22). Accordingly, a combination of the environment temperature and the duration ratio of the parasympathetic nerve activity of the sleeper (P) is stored in the storage unit (22) for each sleeper (P). In this way, the correspondence relationship between the environment temperature and the duration ratio of the parasympathetic nerve activity of the sleeper (P) is stored in the storage unit (22).

### <Step (S15)>

Next, the estimation unit (32) determines whether preparation for estimating the appropriate value (T1) of the environment temperature suitable for the sleeper (P) is completed. In this example, if the number of combinations of the environment temperature and the duration ratio of the parasympathetic nerve activity of the sleeper (P) stored in the storage unit (22) exceeds a predetermined threshold value, the estimation unit (32) determines that the preparation for estimating the appropriate value (T1) of the environment temperature suitable for the sleeper (P) is completed.

If the preparation for estimating the appropriate value (T1) of the environment temperature suitable for the sleeper (P) is completed, the process of step (S17) is performed; if not, the process of step (S16) is performed.

### <Step S16>

If the preparation for estimating the appropriate value (T1) of the environment temperature suitable for the sleeper (P) is not completed, the derivation unit (31) sets the environment temperature to a next environment temperature before the next time the sleeper (P) enters the bed (for example, 30 minutes before the entry to the bed next day).

Specifically, the derivation unit (31) changes the target environment temperature to the next target environment temperature before the next time the sleeper (P) enters the bed. The air conditioning control unit (35) controls the air conditioner (15) so that the environment temperature detected by the environment temperature sensor (11) becomes the target environment temperature (the target environment temperature after the change) set by the derivation unit (31).

Next, the process of step (S12) is performed. Examples of the procedure for setting the environment temperature in step (S16) include the following three patterns.

### <<First pattem>>

In the first pattern, a rule indicating how to change the environment temperature is determined in advance. For example, the rule indicates that the environment temperature is decreased by a predetermined amount (for example, 0.5°C) each time the process of step (S16) is performed. The derivation unit (31) sets an environment temperature (specifically, a target environment temperature) based on the predetermined rule.

### <<Second pattern>>

In the second pattern, the derivation unit (31) in the first step (S16) sets the environment temperature to a temperature lower than the initial value of the environment temperature (the environment temperature set in step (S11)). For example, when the initial value of the environment temperature is 26°C, the derivation unit (31) sets the environment temperature to 25.5°C in the first step (S16).

In addition, in the second pattern, the derivation unit (31) performs the following processing in a second or subsequent step (S16).

The derivation unit (31) determines whether the duration ratio of the parasympathetic nerve activity of the sleeper (P) derived in step (S13) exceeds the previous value (the duration ratio of the parasympathetic nerve activity of the sleeper (P) derived in the previous step (S13)).

If the duration ratio (latest value) of the parasympathetic nerve activity of the sleeper (P) derived in step (S13) exceeds the previous value, the derivation unit (31) sets the environment temperature to a temperature lower than the current temperature. For example, the derivation unit (31) decreases the environment temperature by a predetermined amount (for example, 0.5°C).

On the other hand, if the duration ratio (latest value) of the parasympathetic nerve activity of the sleeper (P) derived in step (S13) does not exceed the previous value, the derivation unit (31) sets the environment temperature to a temperature higher than the current temperature. For example, the derivation unit (31) increases the environment temperature by a predetermined amount (e.g., 1°C). As described above, for example, the amount of increase in the environment temperature in step (S16) may be larger than the amount of decrease in the environment temperature in step (S16).

### <<Third pattern>>

In the third pattern, the derivation unit (31) performs the following processing in the first step (S16).

The derivation unit (31) determines whether the duration ratio of the parasympathetic nerve activity of the sleeper (P) derived in step (S13) exceeds a predetermined standard value.

If the duration ratio (latest value) of the parasympathetic nerve activity of the sleeper (P) derived in step (S13) exceeds the standard value, the derivation unit (31) sets the environment temperature to a temperature lower than the current temperature. For example, the derivation unit (31) decreases the environment temperature by a predetermined amount (for example, 0.5°C).

On the other hand, if the duration ratio (latest value) of the parasympathetic nerve activity of the sleeper (P) derived in step (S13) does not exceed the standard value, the derivation unit (31) sets the environment temperature to a temperature higher than the current temperature. For example, the derivation unit (31) increases the environment temperature by a predetermined amount (e.g., 1°C).

In addition, in the third pattern, the derivation unit (31) performs the same process as the process in the second or subsequent step (S16) of the second pattern in the second or subsequent step (S16). The derivation unit (31) determines whether the duration ratio of the parasympathetic nerve activity of the sleeper (P) derived in step (S13) exceeds the previous value, and changes the environment temperature in accordance with the determination result.

Next, the process of step (S12) is performed.

### <Step S17>

Meanwhile, if the preparation for estimating the appropriate value (T1) of the environment temperature suitable for the sleeper (P) is completed in step (S15), the estimation unit (32) estimates the appropriate value (T1) of the environment temperature suitable for the sleeper (P) based on the correspondence relationship between the environment temperature and the duration ratio of the parasympathetic nerve activity of the sleeper (P) stored in the storage unit (22).

Specifically, the estimation unit (32) detects "an environment temperature associated with a duration ratio of the parasympathetic nerve activity of the sleeper (P) higher than a predetermined ratio threshold" from the combinations of the environment temperature and the duration ratio of the parasympathetic nerve activity of the sleeper (P) stored in the storage unit (22), and estimates the detected environment temperature as the appropriate value (T1) of the environment temperature suitable for the sleeper (P). In this example, the estimation unit (32) estimates the environment temperature associated with the highest duration ratio of the parasympathetic nerve activity of the sleeper (P) as the appropriate value (T1) of the environment temperature suitable for the sleeper (P).

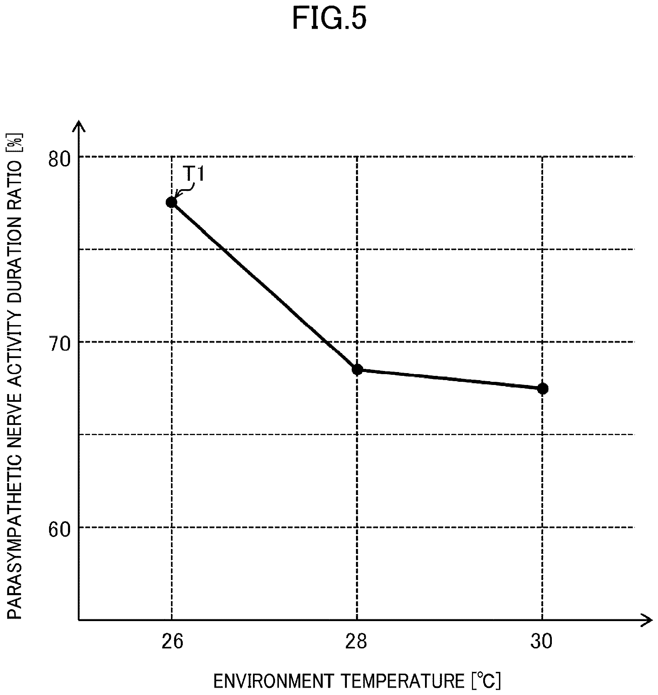

For example, as shown in Fig. 5, if the correspondence relationship between the environment temperature and the duration ratio of the parasympathetic nerve activity of the sleeper (P) is such that "the duration ratio of the parasympathetic nerve activity of the sleeper (P) gradually increases as the environment temperature decreases", the estimation unit (32) estimates the lowest environment temperature corresponding to the highest duration ratio of the parasympathetic nerve activity of the sleeper (P) as the appropriate value (T1) of the environment temperature suitable for the sleeper (P). In the example of Fig. 5, the appropriate value (T1) of the environment temperature suitable for the sleeper (P) is "26°C".

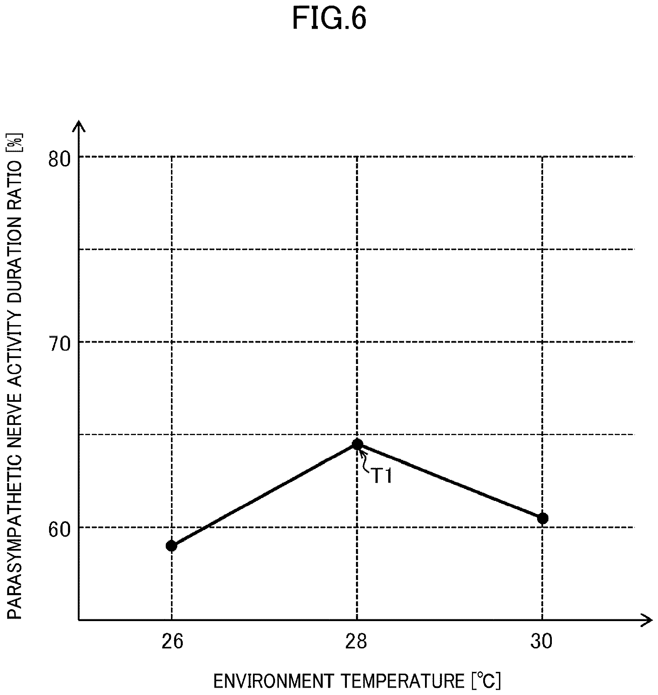

Further, as shown in Fig. 6, if the correspondence relationship between the environment temperature and the duration ratio of the parasympathetic nerve activity of the sleeper (P) is such that "as the environment temperature decreases, the duration ratio of the parasympathetic nerve activity of the sleeper (P) gradually increases, reaches a maximum value, and then gradually decreases", the estimation unit (32) estimates the environment temperature corresponding to the maximum value of the duration ratio of the parasympathetic nerve activity of the sleeper (P) as the appropriate value (T1) of the environment temperature suitable for the sleeper (P). In the example of Fig. 6, the appropriate value (T1) of the environment temperature suitable for the sleeper (P) is "28°C".

### [Air conditioning control]

Next, air conditioning control according to the first embodiment will be described.

The air conditioning control unit (35) sets the "appropriate value (T1) of the environment temperature suitable for the sleeper (P)" estimated by the temperature estimation process as the target environment temperature. Then, the air conditioning control unit (35) controls the air conditioner (15) so that the environment temperature detected by the environment temperature sensor (11) is maintained at the target environment temperature.

Specifically, when the cooling operation is performed by the air conditioner (15), if the environment temperature detected by the environment temperature sensor (11) is higher than the target environment temperature, the air conditioning control unit (35) controls the air conditioner (15) so that the cooling capacity of the air conditioner (15) increases as the difference between the environment temperature and the target environment temperature increases. The air conditioning control unit (35) stops the operation of the air conditioner (15) if the environment temperature detected by the environment temperature sensor (11) is lower than the target environment temperature.

Further, when the heating operation is performed by the air conditioner (15), if the environment temperature detected by the environment temperature sensor (11) is lower than the target environment temperature, the air conditioning control unit (35) controls the air conditioner (15) so that the heating capacity of the air conditioner (15) increases as the difference between the environment temperature and the target environment temperature increases. The air conditioning control unit (35) stops the operation of the air conditioner (15) if the environment temperature detected by the environment temperature sensor (11) is higher than the target environment temperature.

### [Effect of First Embodiment]

As described above, in the air conditioning system (10) of the first embodiment, the derivation unit (31) determines the stability of the parasympathetic nerve activity of the sleeper (P) for each environment temperature, and derives the correspondence relationship between the environment temperature and the stability of the parasympathetic nerve activity of the sleeper (P). Such a configuration makes it possible to estimate the appropriate value (T1) of the environment temperature suitable for the sleeper (P).

Further, in the air conditioning system (10) of the first embodiment, the air conditioning control unit (35) controls the air conditioner (15) based on the appropriate value (T1) of the environment temperature estimated by the temperature estimation device (30) (specifically, the estimation unit (32)). Such a configuration makes it possible to appropriately control the air conditioner (15) based on the appropriate value (T1) of the environment temperature suitable for the sleeper (P).

### (Second Embodiment)

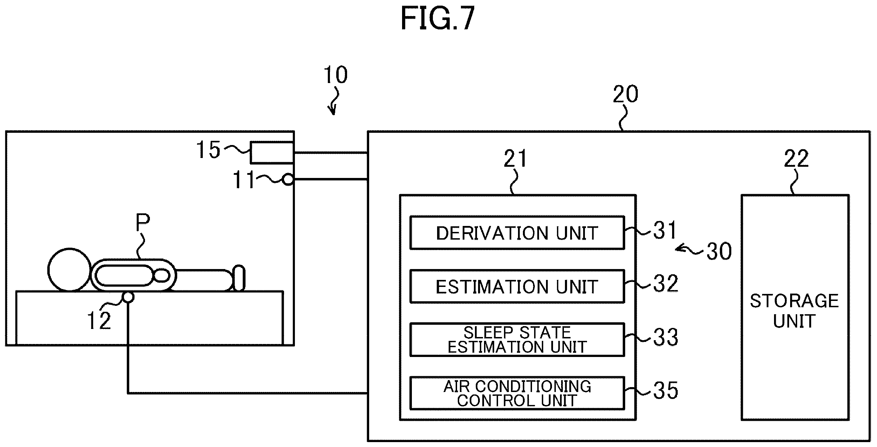

Fig. 7 illustrates a configuration of the air conditioning system (10) according to the second embodiment. The air conditioning system (10) of the second embodiment differs from the air conditioning system (10) of the first embodiment in the control unit (21) of the air conditioning control device (20). Other examples of the air conditioning system (10) of the second embodiment are the same as configurations of the air conditioning system (10) of the first embodiment.

In the second embodiment, the control unit (21) includes a sleep state estimation unit (33) in addition to the configuration of the control unit (21) of the first embodiment.

The sleep state estimation unit (33) estimates the sleep state of the sleeper (P) based on a biological signal (in this example, a heartbeat signal) of the sleeper (P) detected by the biological sensor (12). Specifically, the sleep state estimation unit (33) estimates the sleep depth of the sleeper (P). The sleep state estimation unit (33) estimates sleep onset, a sleep cycle, REM sleep, and non-REM sleep of the sleeper (P). A well-known estimation technique can be adopted for the estimation of the sleep state.

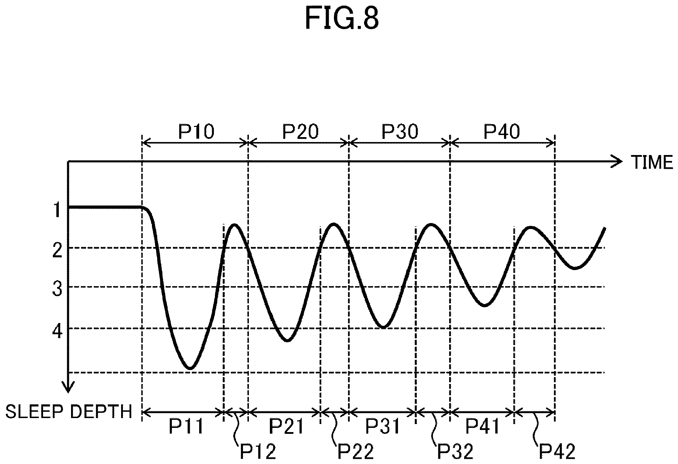

As shown in Fig. 8, the sleep of the sleeper (P) has a cycle, and a non-REM sleep period in which the sleep depth of the sleeper (P) is relatively deep and a REM sleep period in which the sleep depth of the sleeper (P) is relatively shallow alternately appear. In the example of Fig. 8, four cycle periods (first to fourth cycle periods (P10 to P40)) appear. The first cycle period (P10) is a cycle period that first appears after the sleeper (P) falls asleep. In the first cycle period (P10), a first non-REM sleep period (P11) is followed by a first REM sleep period (P12). Similarly, in the second, third, and fourth cycle periods (P20, P30, P40), the second, third, and fourth non-REM sleep periods (P21, P31, P41) are followed by the second, third, and fourth REM sleep periods (P22, P32, P42). The sleep depth of the sleeper (P) in the first to fourth non-REM sleep periods (P11 to P41) gradually becomes shallower toward the awakening of the sleeper (P).

Further, in the second embodiment, the derivation unit (31) determines the stability of the parasympathetic nerve activity of the sleeper (P) based on the parasympathetic nerve activity of the sleeper (P) in the non-REM sleep periods (P11, P21, P31, P41) of the sleeper (P).

### [Temperature estimation process]

The temperature estimation process of the second embodiment differs from the temperature estimation process (see Fig. 2) of the first embodiment in the process of step (S13). Other processes (steps (S 11, S12, S14 to S17)) of the temperature estimation process of the second embodiment are the same as those of the temperature estimation process of the first embodiment.

### <Step (S13)>

In step (S13) of the second embodiment, the derivation unit (31) extracts biological signals corresponding to the non-REM sleep periods (P11, P21, P31, P41) of the sleeper (P) from the biological signals of the sleeper (P) collected in step (S12). Then, the derivation unit (31) derives the duration ratio of the parasympathetic nerve activity of the sleeper (P) based on the extracted biological signals (biological signals corresponding to the non-REM sleep periods (P11, P21, P31, P41) of the sleeper (P)).

### [Effect of Second Embodiment]

As described above, in the air conditioning system (10) of the second embodiment, the derivation unit (31) determines the stability of the parasympathetic nerve activity of the sleeper (P) based on the parasympathetic nerve activity of the sleeper (P) in the non-REM sleep periods (P11, P21, P31, P41) of the sleeper (P). Such a configuration makes it possible to accurately determine the stability of the parasympathetic nerve activity of the sleeper (P) while avoiding the REM sleep periods (P12, P22, P32, P42), in which the autonomic nerves of the sleeper (P) tend to be unstable. Accordingly, it is possible to accurately estimate the appropriate value (T1) of the environment temperature suitable for the sleeper (P).

### (Third Embodiment)

In the air conditioning system (10) of the third embodiment, the control unit (21) of the air conditioning control device (20) of the second embodiment is different from that of the air conditioning control device (20) of the second embodiment. Other examples of the air conditioning system (10) of the third embodiment are the same as configurations of the air conditioning system (10) of the second embodiment.

In the third embodiment, the derivation unit (31) derives the correspondence relationship between the environment temperature and the stability of the parasympathetic nerve activity of the sleeper (P) for each sleep stage of the sleeper (P). The estimation unit (32) estimates the appropriate value (T1) of the environment temperature suitable for the sleeper (P) based on the correspondence relationship between the environment temperature and the stability of the parasympathetic nerve activity of the sleeper (P) for each sleep stage of the sleeper (P). The air conditioning control unit (35) controls the air conditioner (15) based on the appropriate value (T1) of the environment temperature estimated for each sleep stage of the sleeper (P).

### [Temperature estimation process]

The temperature estimation process of the third embodiment differs from the temperature estimation process of the second embodiment in the processes of step (S13), step (S14), and step (S17). Other processes of the temperature estimation process of the third embodiment (steps (S11, S12, S15, S16)) are the same as those of the temperature estimation process of the second embodiment. The cycle periods (P10 to P40) are examples of the sleeping stages (elapsed stages).

### <Step (S13)>

In step (S13) of the third embodiment, the derivation unit (31) derives the duration ratio of the parasympathetic nerve activity of the sleeper (P) for each of the cycle periods (P10 to P40). Specifically, the derivation unit (31) selects any one of the cycle periods (P10 to P40) as a cycle period to be processed. Next, the derivation unit (31) extracts a biological signal in the cycle period to be processed from the biological signals of the sleeper (P) collected in step (S12). Next, the derivation unit (31) derives the duration ratio of the parasympathetic nerve activity of the sleeper (P) in the cycle period to be processed, based on the extracted biological signal. Next, the derivation unit (31) selects a next cycle period to be processed from the cycle periods (P10 to P40). In this manner, the cycle periods (P10 to P40) are sequentially selected one by one and processed, whereby the duration ratio of the parasympathetic nerve activity of the sleeper (P) is derived for each of the cycle periods (P10 to P40).

### <Step (S14)>

In step (S14) of the third embodiment, the derivation unit (31) stores identification information for identifying the sleeper (P), the environment temperature set in step (S11) (or step (S16)), the duration ratio of the parasympathetic nerve activity of the sleeper (P) derived for each of the cycle periods (P10 to P40) in step (S13), and period information indicating the cycle period in which the duration ratio of the parasympathetic nerve activity of the sleeper (P) is derived, in association with each other in the storage unit (22). Thus, a combination of the cycle period, the environment temperature, and the duration ratio of the parasympathetic nerve activity of the sleeper (P) is stored in the storage unit (22) for each sleeper (P). In this manner, the correspondence relationship among the "cycle period", the "environment temperature", and the "duration ratio of the parasympathetic nerve activity of the sleeper (P)" is stored in the storage unit (22).

### <Step (S17)>

In step (S17) of the third embodiment, the derivation unit (31) estimates the appropriate value (T1) of the environment temperature suitable for the sleeper (P) for each of the cycle periods (P10 to P40) based on the correspondence relationship among the "cycle period", the "environment temperature", and the "duration ratio of the parasympathetic nerve activity of the sleeper (P)" stored in the storage unit (22).

Specifically, the estimation unit (32) selects any one of the cycle periods (P10 to P40) as a cycle period to be processed. Next, the estimation unit (32) extracts combinations of the environment temperature and the duration ratio of the parasympathetic nerve activity of the sleeper (P) that are associated with the cycle period to be processed, from the storage unit (22). Next, the estimation unit (32) detects "the environment temperature associated with a duration ratio of the parasympathetic nerve activity of the sleeper (P) higher than a predetermined ratio threshold" from the extracted combinations, and estimates the detected environment temperature as the appropriate value (T1) of the environment temperature suitable for the sleeper (P) in the cycle period to be processed. In this example, the estimation unit (32) estimates the environment temperature associated with the highest duration ratio of the parasympathetic nerve activity of the sleeper (P) as the appropriate value (T1) of the environment temperature suitable for the sleeper (P) in the cycle period to be processed. In this manner, the cycle periods (P10 to P40) are sequentially selected one by one and processed, whereby the appropriate value (T1) of the environment temperature suitable for the sleeper (P) is estimated for each of the cycle periods (P10 to P40).

### [Effect of Third Embodiment]

As described above, in the air conditioning system (10) according to the third embodiment, the derivation unit (31) derives the correspondence relationship between the environment temperature and the stability of the parasympathetic nerve activity of the sleeper (P) for each sleep stage of the sleeper (P). Such a configuration makes it possible to estimate the appropriate value (T1) of the environment temperature suitable for the sleeper (P) for each sleep stage of the sleeper (P).

In the foregoing description, as examples of the sleeping stages (elapsed stages) of the sleeper (P), the sleeping cycle periods (P10 to P40) of the sleeper (P) have been described. However, this is not a limitation. For example, the derivation unit (31) may derive the correspondence relationship between the environment temperature and the stability of the parasympathetic nerve activity of the sleeper (P) for each elapsed time from the entry to the bed (or sleep onset) of the sleeper (P). For example, the estimation unit (32) may estimate the appropriate value (T1) of the environment temperature suitable for the sleeper (P) based on the correspondence relationship between the environment temperature and the stability of the parasympathetic nerve activity of the sleeper (P) for each elapsed time from the entry to the bed (or sleep onset) of the sleeper (P). For example, the air conditioning control unit (35) may control the air conditioner (15) based on the appropriate value (T1) of the environment temperature estimated for each elapsed time from the entry to the bed (or sleep onset) of the sleeper (P).

### (Fourth Embodiment)

The air conditioning system (10) of the fourth embodiment differs from the air conditioning system (10) of the second embodiment in the air conditioning control unit (35) of the air conditioning control device (20). Other configurations of the air conditioning system (10) of the fourth embodiment are the same as those of the air conditioning system (10) of the second embodiment.

In the fourth embodiment, the air conditioning control unit (35) controls the air conditioner (15) so that the environment temperature becomes the appropriate value (T1) of the environment temperature estimated by the estimation unit (32). Thereafter, if a predetermined condition is satisfied, the air conditioning control unit (35) controls the air conditioner (15) so that the environment temperature becomes higher than the appropriate value (T 1). The condition includes at least one of the following first to fourth conditions.

### [First condition]

The first condition is a condition that the first REM sleep period (P12) starts after the sleeper (P) falls asleep. In this example, the air conditioning control unit (35) determines whether the first REM sleep period (P12) has started after the sleeper (P) fell asleep, based on the estimation result by the sleep state estimation unit (33). When the first REM sleep period (P12) starts after the sleeper (P) has fallen asleep, the air conditioning control unit (35) determines that the first condition is satisfied. If the first condition is satisfied, the air conditioning control unit (35) changes the target environment temperature to a temperature higher than the appropriate value (T1) of the environment temperature.

In the first non-REM sleep period (P11) of the first cycle period (P10), the sleep depth of the sleeper (P) is deep, and it is considered that the effect of promoting deep sleep by decreasing the body temperature of the sleeper (P) is large. However, after the second cycle period (P20), the proportion of the REM sleep period with respect to the cycle period increases, and the time in which the sleeper (P) becomes sensitive to the environment temperature increases. Therefore, it is considered that the effect of reducing the risk of the sleeper (P) getting chilled while asleep by increasing the body temperature of the sleeper (P) is greater than the effect of promoting deep sleep by decreasing the body temperature of the sleeper (P).

Therefore, by controlling the air conditioner (15) so that the environment temperature becomes higher than the appropriate value (T1) after the first REM sleep period (P12) starts after the sleeper (P) falls asleep, excessive cooling of the sleeper (P) can be suppressed in the periods after the first REM sleep period (P12), and the risk of the sleeper (P) getting chilled while asleep can be reduced.

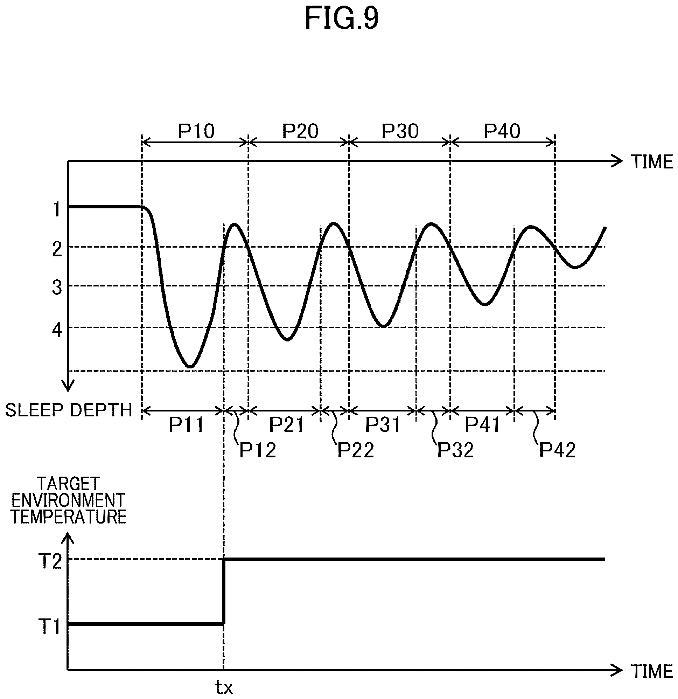

For example, as shown in Fig. 9, at a time point (tx) at which the first REM sleep period (P12) starts, the air conditioning control unit (35) changes the target environment temperature to a temperature (T2) higher than the appropriate value (T 1) of the environment temperature. The target environment temperature after the change is set to a temperature at which the risk of the sleeper (P) getting chilled while asleep can be reduced. Such a temperature can be set by experiments or machine learning. The difference between the target environment temperature after the change and the target environment temperature before the change (the appropriate value (T1) of the environment temperature) is, for example, 1°C.

### [Second condition]

The second condition is that the amount of decrease in the stability of the parasympathetic nerve activity of the sleeper (P) exceeds a predetermined reference amount after the sleeper (P) has fallen asleep. In this example, after the sleeper (P) has fallen asleep, the derivation unit (31) derives, for each predetermined monitoring period, the duration ratio of the parasympathetic nerve activity of the sleeper (P) in the monitoring period based on the biological signal detected by the biological sensor (12). The air conditioning control unit (35) derives an amount of decrease in the duration ratio of the parasympathetic nerve activity of the sleeper (P) by subtracting the duration ratio of the parasympathetic nerve activity of the sleeper (P) in the latest monitoring period derived by the derivation unit (31) from the duration ratio of the parasympathetic nerve activity of the sleeper (P) in the previous monitoring period derived by the derivation unit (31). Next, the air conditioning control unit (35) determines whether the amount of decrease in the duration ratio of the parasympathetic nerve activity of the sleeper (P) exceeds a predetermined reference amount. If the amount of decrease in the duration ratio of the parasympathetic nerve activity of the sleeper (P) falls below the reference amount, the air conditioning control unit (35) determines that the second condition is satisfied. If the second condition is satisfied, the air conditioning control unit (35) changes the target environment temperature to a temperature higher than the appropriate value (T1) of the environment temperature.

If the sleeper (P) is excessively cooled after the sleeper (P) enters the bed, it is considered that the stability of the parasympathetic nerve activity of the sleeper (P) decreases. Therefore, by controlling the air conditioner (15) so that the environment temperature becomes higher than the appropriate value (T1) after the amount of decrease in the stability of the parasympathetic nerve activity of the sleeper (P) exceeds the reference amount after the sleeper (P) has fallen asleep, it is possible to suppress excessive cooling of the sleeper (P).

### [Third condition]

The third condition is a condition that a predetermined time elapses after the sleeper (P) enters the bed. In this example, the air conditioning control unit (35) detects whether the sleeper (P) has entered the bed based on the presence or absence of a biological signal output from the biological sensor (12). When a predetermined time elapses after the sleeper (P) enters the bed, the air conditioning control unit (35) determines that the third condition is satisfied. If the third condition is satisfied, the air conditioning control unit (35) changes the target environment temperature to a temperature higher than the appropriate value (T1) of the environment temperature.

The body temperature of the sleeper (P) tends to gradually decrease after the sleeper (P) has fallen asleep, and to then gradually increase toward awakening of the sleeper (P). If the environment temperature is too low during the period in which the body temperature gradually increases toward the awakening of the sleeper (P), the sleeper (P) becomes too cold.

For example, the predetermined time in the third condition may be set to a time period from the time point at which the sleeper (P) enters the bed to the time point at which the body temperature of the sleeper (P) starts to increase toward the awakening of the sleeper (P). In this case, by controlling the air conditioner (15) so that the environment temperature becomes higher than the appropriate value (T1) upon the elapse of a predetermined time after the sleeper (P) enters the bed, it is possible to suppress excessive cooling of the sleeper (P) in a period in which the body temperature gradually increases toward the awakening of the sleeper (P). Such a predetermined time can be set by experiment or machine learning.

Alternatively, the predetermined time in the third condition may be set to a time period from the time point at which the sleeper (P) enters the bed to the time point at which the first REM sleep period (P12) starts. In this case, by controlling the air conditioner (15) so that the environment temperature becomes higher than the appropriate value (T1) upon the elapse of a predetermined time after the sleeper (P) enters the bed, excessive cooling of the sleeper (P) can be suppressed in the periods after the first REM sleep period (P12), and the risk of the sleeper (P) getting chilled while asleep can be reduced. Such a predetermined time can be set by experiment or machine learning.

### [Fourth condition]

The fourth condition is a condition that a predetermined time elapses after the sleeper (P) falls asleep. In this example, the air conditioning control unit (35) detects sleep onset of the sleeper (P) based on the estimation result by the sleep state estimation unit (33). When the predetermined time elapses after the sleeper (P) falls asleep, the air conditioning control unit (35) determines that the fourth condition is satisfied. If the fourth condition is satisfied, the air conditioning control unit (35) changes the target environment temperature to a temperature higher than the appropriate value (T1) of the environment temperature.

For example, the predetermined time in the fourth condition may be set to a time period from the time point at which the sleeper (P) falls asleep to the time point at which the body temperature of the sleeper (P) starts to increase toward awakening of the sleeper (P). In this case, by controlling the air conditioner (15) so that the environment temperature becomes higher than the appropriate value (T1) upon the elapse of the predetermined time after the sleeper (P) has fallen asleep, it is possible to suppress excessive cooling of the sleeper (P) in a period in which the body temperature gradually increases toward the awakening of the sleeper (P). Such a predetermined time can be set by experiment or machine learning.

Alternatively, the predetermined time in the fourth condition may be set to a time period from the time point at which the sleeper (P) falls asleep to the time point at which the first REM sleep period (P12) starts. In this case, by controlling the air conditioner (15) so that the environment temperature becomes higher than the appropriate value (T1) upon the elapse of a predetermined time after the sleeper (P) has fallen asleep, excessive cooling of the sleeper (P) can be suppressed in the periods after the first REM sleep period (P12), and the risk of the sleeper (P) getting chilled while asleep can be reduced. Such a predetermined time can be set by experiment or machine learning.

### [Effect of Fourth Embodiment]

As described above, in the air conditioning system (10) of the fourth embodiment, the air conditioning control unit (35) controls the air conditioner (15) so that the environment temperature becomes the appropriate value (T1), and then controls the air conditioner (15) so that the environment temperature becomes higher than the appropriate value (T1) if a predetermined condition is satisfied. Such a configuration makes it possible to suppress excessive cooling of the sleeper (P) after the predetermined condition is satisfied. In this example, it is possible to suppress excessive cooling of the sleeper (P) after at least one of the first to fourth conditions is satisfied.

### (Fifth Embodiment)

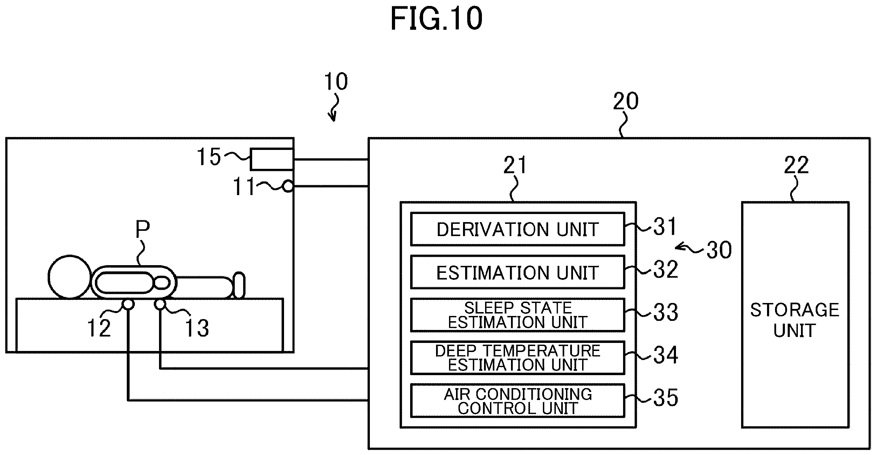

Fig. 10 illustrates a configuration of the air conditioning system (10) according to a fifth embodiment. The air conditioning system (10) of the fifth embodiment differs from the air conditioning system (10) of the second embodiment in the control unit (21) of the air conditioning control device (20). Other configurations of the air conditioning system (10) of the fifth embodiment are the same as those of the air conditioning system (10) of the second embodiment.

The air conditioning system (10) of the fifth embodiment is provided with a body temperature sensor (13) in addition to the environment temperature sensor (11) and the biological sensor (12). The body temperature sensor (13) detects the body temperature of the sleeper (P).

In the fifth embodiment, the control unit (21) includes a deep temperature estimation unit (34) in addition to the configuration of the control unit (21) of the second embodiment.

The deep temperature estimation unit (34) estimates the deep temperature of the sleeper (P). In this example, the deep temperature estimation unit (34) estimates the deep temperature of the sleeper (P) based on the body temperature (surface temperature) of the sleeper (P) detected by the body temperature sensor (13).

In the fifth embodiment, the air conditioning control unit (35) controls the air conditioner (15) so that the environment temperature changes at a second time point (t2) before a first time point (t1) at which the deep temperature of the sleeper (P) is to be changed.

Examples of the first time point (t1) at which the deep temperature of the sleeper (P) is to be changed include a time point at which the first REM sleep period (P12) starts after the sleeper (P) falls asleep, a time point at which a predetermined time elapses after the sleeper (P) enters the bed, and a time point at which a predetermined time elapses after the sleeper (P) falls asleep. The first time point (t1) at which the deep temperature of the sleeper (P) is to be changed can be set (predicted) in advance by experiment or machine learning.

Further, the second time point (t2) is earlier than the first time point (t1) by a predetermined time (tp). The time (tp) is a time corresponding to a time difference before a change in the environment temperature affects the deep temperature of the sleeper (P). For example, the time (tp) includes a first time corresponding to a time difference from when the environment temperature changes to when the surface temperature of the sleeper (P) changes, and a second time corresponding to a time difference from when the surface temperature of the sleeper (P) changes to when the deep temperature of the sleeper (P) changes. The time (tp) can be set by experiment or machine learning. For example, the first time can be set based on the load of the space in which the sleeper (P) is present.

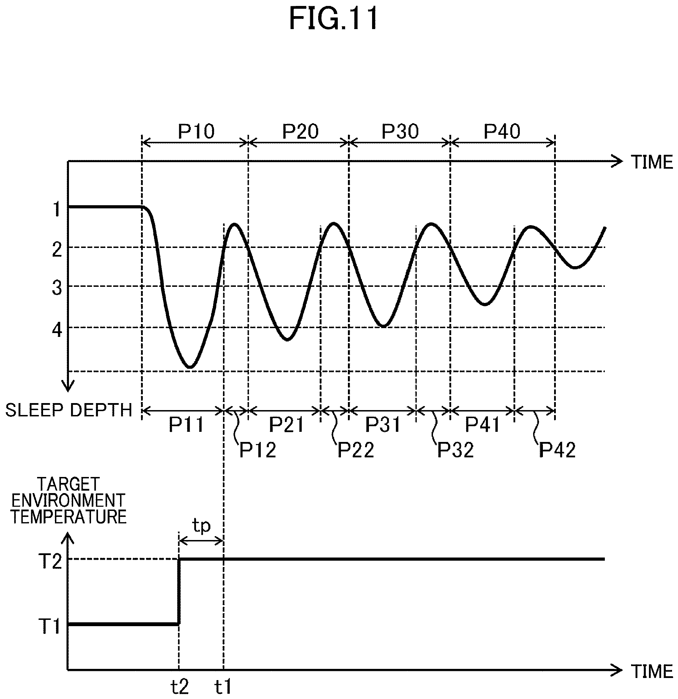

For example, as shown in Fig. 11, if the first time point (t1) is "a time point at which the first REM sleep period (P12) starts after the sleeper (P) falls asleep", the air conditioning control unit (35) changes the target environment temperature to a temperature (T2) higher than the appropriate value (T1) of the environment temperature at the second time point (t2) which is earlier by the time (tp) than the time point at which the first REM sleep period (P12) starts after the sleeper P falls asleep.

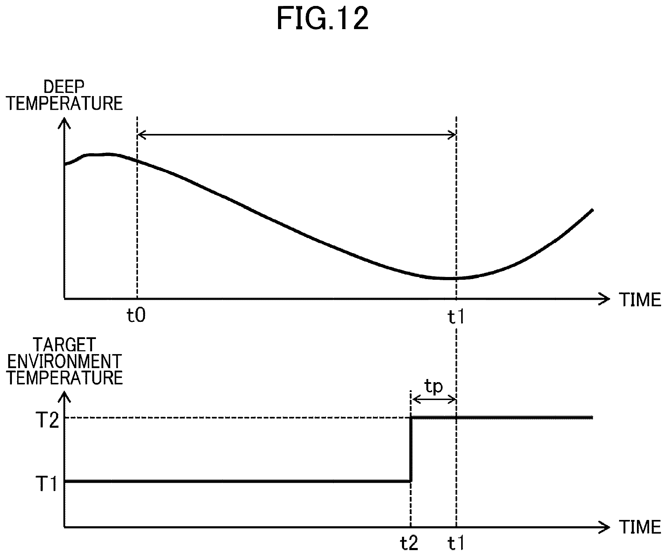

As shown in Fig. 12, if the first time point (t1) is "a time point at which a predetermined time elapses after the sleeper (P) falls asleep", the air conditioning control unit (35) changes the target environment temperature to the temperature (T2) higher than the appropriate value (T1) of the environment temperature at the second time point (t2) which is earlier by the time (tp) than the time point (t1) at which the predetermined time has elapsed from the time point (t0) at which the sleeper (P) fell asleep.

### [Effect of Fifth Embodiment]

As described above, in the air conditioning system (10) of the fifth embodiment, the air conditioning control unit (35) controls the air conditioner (15) so that the environment temperature changes at the second time point (t2) before the first time point (t1) at which the deep temperature of the sleeper (P) is to be changed. Such a configuration makes it possible to control the environment temperature in consideration of the time difference before a change in the environment temperature affects the deep temperature of the sleeper (P). This makes it possible to appropriately control the deep temperature of the sleeper (P).

### (Other Embodiments)

In the foregoing description, as an example of the stability of the parasympathetic nerve activity of the sleeper (P), the duration ratio of the parasympathetic nerve activity of the sleeper (P) derived based on the time-series data signal of the peak interval (RR interval) of the heartbeat waveform has been described. However, this is not a limitation. For example, the stability of the parasympathetic nerve activity of the sleeper (P) may be a duration ratio of the parasympathetic nerve activity of the sleeper (P) derived based on a time-series data signal of a coefficient of variance of R-R interval (CVRR). The coefficient of variance of R-R interval is an index value corresponding to the ratio of a standard deviation to an average value of an interval between R waves (RRI: R-R interval), and is an index value used as an index of mental stress.

While embodiments and modifications have been described, it will be understood that various changes in form and details may be made therein without departing from the spirit and scope of the claims. The above-described embodiments and modifications may be appropriately combined or replaced as long as the functions of the object of the present disclosure are not impaired.

### Industrial Applicability

As described above, the present disclosure is useful as a temperature estimation device, an air conditioning control device, and an air conditioning system.

### Reference Signs List

- 10: Air conditioning system
- 11: Environment temperature sensor
- 12: Biological sensor
- 13: Body temperature sensor
- 15: Air conditioner
- 20: Air conditioning control device
- 21: Control unit
- 22: Storage unit
- 30: Temperature estimation device
- 31: Derivation unit
- 32: Estimation unit
- 33: Sleep state estimation unit
- 34: Deep temperature estimation unit
- 35: Air conditioning control unit
- P: Sleeper

## Claims

1. A temperature estimation device comprising:
a derivation unit (31) configured to determine a stability of a parasympathetic nerve activity of a sleeper (P) for each environment temperature, and to derive a correspondence relationship between the environment temperature and the stability of the parasympathetic nerve activity of the sleeper (P); and
an estimation unit (32) configured to estimate an appropriate value (T1) of the environment temperature suitable for the sleeper (P) based on the correspondence relationship derived by the derivation unit (31).

2. The temperature estimation device according to claim 1, wherein
the derivation unit (31) determines the stability of the parasympathetic nerve activity of the sleeper (P) based on the parasympathetic nerve activity of the sleeper (P) in a non-REM sleep period (P11, P21, P31, P41) of the sleeper (P).

3. The temperature estimation device according to claim 1 or 2, wherein
the derivation unit (31) derives the correspondence relationship between the environment temperature and the stability of the parasympathetic nerve activity of the sleeper (P) for each sleep stage of the sleeper (P).

4. An air conditioning control device for controlling an air conditioner (15) that performs air conditioning of a space in which a sleeper (P) is present, the air conditioning control device comprising:
the temperature estimation device according to any one of claims 1 to 3; and
an air conditioning control unit (35) configured to control the air conditioner (15) based on the appropriate value (T1) of the environment temperature estimated by the temperature estimation device.

5. The air conditioning control device according to claim 4, wherein
the air conditioning control unit (35) controls the air conditioner (15) so that the environment temperature becomes the appropriate value (T1), and then, if a predetermined condition is satisfied, controls the air conditioner (15) so that the environment temperature becomes higher than the appropriate value (T1).

6. The air conditioning control device according to claim 5, wherein
the condition includes at least one of a first condition that a first REM sleep period (P12) starts after the sleeper (P) falls asleep, a second condition that an amount of decrease in the stability of the parasympathetic nerve activity of the sleeper (P) exceeds a predetermined reference amount after the sleeper (P) falls asleep, a third condition that a predetermined time elapses after the sleeper (P) enters a bed, or a fourth condition that a predetermined time elapses after the sleeper (P) falls asleep.

7. The air conditioning control device according to claim 4, wherein
the air conditioning control unit (35) controls the air conditioner (15) so that the environment temperature changes at a second time point (t2) before a first time point (t1) at which a deep temperature of the sleeper (P) is to be changed.

8. An air conditioning system comprising:
an air conditioner (15) configured to perform air conditioning of a space in which a sleeper (P) is present; and
the air conditioning control device according to any one of claims 4 to 7 configured to control the air conditioner (15).
